# EUROPEAN PATENT APPLICATION

(11) **EP 4 385 494 A1**
(43) Date of publication of application: **19.06.2024**
(21) Application number: 21840768.2
(22) Date of filing: 25.10.2021
(51) Int. Cl.: A61K 9/00, A61K 38/47, A61K 45/06, A61M 37/00

(54) **MICRONEEDLE PATCH**

(30) Priority: 12.08.2021 KR 20210106705
(71) Applicant: Feroka Inc., Seoul 04784 (KR)
(72) Inventor: LEE, Jae Joon, Seoul 02793 (KR); JEON, Yi Seul, Seoul 08799 (KR)
(74) Representative: Hoffmann Eitle
(86) International application number: PCT/KR2021/015029
(87) International publication number: WO 2023/017907

(57) **Abstract**

A microneedle patch includes a base, and a microneedle disposed on a surface of the base and including a base material, an effective material, and an activity promoting material.

## Description

### TECHNICAL FIELD

The present disclosure relates to a microneedle patch.

### BACKGROUND

Injection of drugs into human body has traditionally been done by needle syringes, but the needle syringes cause great pain. Therefore, a non-invasive drug injection method has also been developed, but there is a problem that amount of drug required is too large compared to amount of drug injected.

To solve this problem, a lot of research has been done on a drug delivery system (DDS), which may be further advanced with a development of nanotechnology.

Unlike conventional injection needles, microneedles have characteristics of less pain and no trauma when penetrating the skin. In addition, since the microneedles have to penetrate the stratum corneum of the skin, a certain degree of hardness is required, and an appropriate length may be required for physiologically active substance to reach the epidermal or dermal layer of the skin. In addition, in order for the physiologically active substances of hundreds of microneedles to be effectively delivered into the skin, the skin permeability of the microneedles must be high and maintained for a certain period of time until dissolution after being inserted into the skin.

Accordingly, interest in a microneedle capable of delivering a drug in a precise amount and accurately setting a target position is increasing.

### DESCRIPTION OF EMBODIMENTS

### TECHNICAL PROBLEM

The present disclosure may provide a microneedle patch capable of effectively delivering a preset amount of an effective material to a target position.

### TECHNICAL SOLUTION TO PROBLEM

A microneedle patch according to an embodiment of the present disclosure includes a base, and a microneedle disposed on a surface of the base, and including a base material, an effective material and an activity promoting material.

### ADVANTAGEOUS EFFECTS OF DISCLOSURE

A microneedle patch according to an embodiment of the present disclosure includes an effective material and an activity promoting material so that the effective material may be delivered to a body quickly and effectively.

A microneedle patch according to an embodiment of the present disclosure has a multi-layer structure to set decomposition rate in vivo of each layer differently, so that effective materials included in each layer may have different activation times.

### BRIEF DESCRIPTION OF DRAWINGS

FIG. 1 is a perspective view illustrating a microneedle patch according to an embodiment of the present disclosure.
FIG. 2 is a cross-sectional view illustrating the microneedle patch of FIG. 1.
FIG. 3 is an enlarged view of a portion of FIG. 2.
FIG. 4 is a diagram illustrating a process in which the microneedle patch of FIG. 2 is attached and a drug is delivered.
FIGS. 5 to 16 is diagrams illustrating a modified example of FIG. 3.
FIG. 17 is a view illustrating a microneedle patch according to another embodiment of the present disclosure.

### BEST MODE

The microneedle patch according to an embodiment of the present disclosure includes a base; and
a microneedle disposed on a surface of the base and includes a base material, an effective material, and an activity promoting material.

In the microneedle patch according to an embodiment of the present disclosure, the activity promoting material may decompose an extracellular matrix (ECM) to increase absorption rate of the effective material.

In the microneedle patch according to an embodiment of the present disclosure, the activity promoting material may be activated before the effective material.

In the microneedle patch according to an embodiment of the present disclosure, the activity promoting material may be disposed on an outside of the microneedle.

In the microneedle patch according to an embodiment of the present disclosure, the activity promoting material may be disposed so that concentration increases from a center of the microneedle toward an outside.

The microneedle patch according to an embodiment of the present disclosure may further include a coating layer disposed on an outside of the microneedle, and including the activity promoting material.

In the microneedle patch according to an embodiment of the present disclosure, the microneedle includes a first base material and the effective material, the coating layer includes a second base material different from the first base material and the activity promoting material, and
the second base material may have a faster decomposition rate than the first base material.

In the microneedle patch according to an embodiment of the present disclosure, at least one of mass per unit volume, moles per unit volume, and volume per unit volume of the activity promoting material may be smaller than that of the effective material.

In the microneedle patch according to an embodiment of the present disclosure, the activity promoting material may be hyaluronidase.

In the microneedle patch according to an embodiment of the present disclosure, the microneedle may include a first needle portion including a first base material, a first effective material and the activity promoting material, and a second needle portion disposed between the base and the first needle portion, and including a second base material, a second effective material and the activity promoting material.

In the microneedle patch according to an embodiment of the present disclosure, the microneedle includes a first needle portion including a first base material, a first effective material and the activity promoting material, and a second needle portion disposed between the base and the first needle portion, and including a second base material and the activity promoting material, and
the second base material may be a faster decomposition rate than the first base material.

In the microneedle patch according to an embodiment of the present disclosure, the microneedle includes a first needle portion including a first base material, a first effective material and the activity promoting material, and a second needle portion disposed between the base and the first needle portion, and including a second base material and a second effective material, and
the first base material may have a faster decomposition rate than the second base material.

Other aspects, features and advantages other than those described above will become apparent from the following detailed description, claims and drawings for carrying out the disclosure.

### DETAILED DESCRIPTION

Since the present disclosure can apply various transformations and can have various embodiments, specific embodiments are illustrated in the drawings and described in detail in the description of the disclosure. However, this is not intended to limit the present disclosure to specific embodiments, and it should be understood to include all modifications, equivalents and substitutes included in the spirit and scope of the present disclosure. In the description of the present disclosure, even though shown in other embodiments, the same identification numbers are used for the same components.

Hereinafter, embodiments of the present disclosure will be described in detail with reference to the accompanying drawings, and the same or corresponding components will be denoted by the same reference numerals when described with reference to the accompanying drawings, and thus their descriptions that are already provided will be omitted.

While such terms as "first," "second," etc., are used only to distinguish one component from another, and such components must not be limited by these terms.

The singular expression also includes the plural meaning as long as it is not inconsistent with the context.

The terms "comprises," "includes," or "has" used herein specify the presence of stated features or components, but do not preclude the presence or addition of one or more other features or components.

In the drawings, the size of the components may be exaggerated or reduced for convenience of description. For example, since the size and thickness of each component shown in the drawings are arbitrarily indicated for convenience of description, the present disclosure is not necessarily limited to the illustrated bar.

In the following embodiments, the x-axis, the y-axis, and the z-axis are not limited to three axes on a Cartesian coordinate system, and may be interpreted in a broad sense including them. For example, the x-axis, y-axis, and z-axis may be orthogonal to each other, but may refer to different directions that are not orthogonal to each other.

In cases where certain embodiments may be implemented otherwise, a specific process sequence may be performed different from the described sequence. For example, two processes described in succession may be performed substantially simultaneously, or may be performed in an order opposite to the order described.

The terms used in the present application are only used to describe specific embodiments, and are not intended to limit the present disclosure. In the present application, terms such as "comprise" or "have" are intended to designate that a feature, number, step, operation, component, part, or combination thereof described in the specification exists, but one or more other features It should be understood that this does not preclude the existence or addition of numbers, steps, operations, components, parts, or combinations thereof.

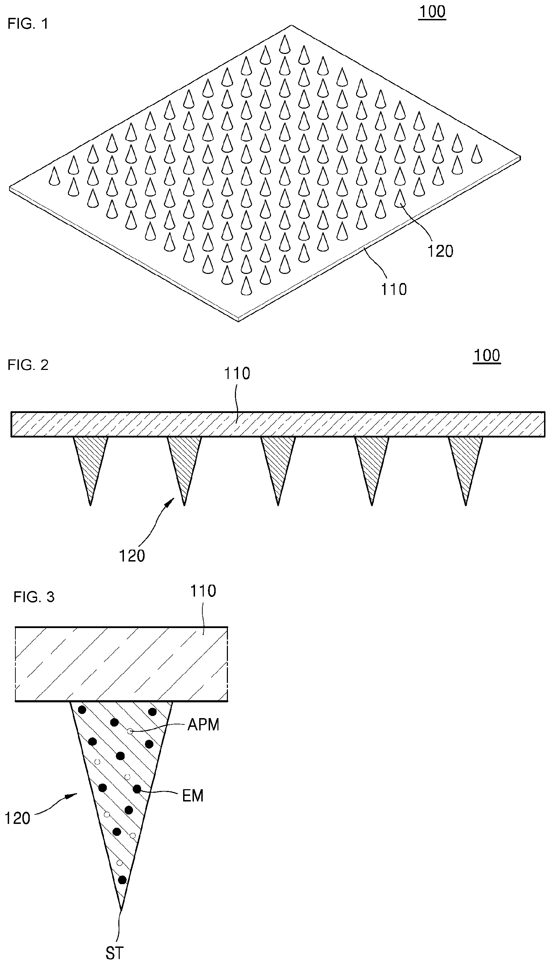

FIG. 1 is a perspective view illustrating a microneedle patch 100 according to an embodiment of the present disclosure, FIG. 2 is a cross-sectional view illustrating the microneedle patch 100 of FIG. 1, and FIG. 3 is an enlarged view of a portion of FIG. 2.

Referring to FIGS. 1 to 3, a microneedle patch 100 according to an embodiment of the present disclosure may include a base 110 and a microneedle 120.

The base 110 may support a plurality of microneedles 120. A shape of the base 110 is not particularly limited, and one surface may have a flat shape so that the plurality of microneedles 120 may be disposed thereon. In a state in which the microneedle patch 100 is disposed on a user's skin, one surface of the base 110 may be in contact with the skin, and the other surface may be exposed to an outside.

The base 110 may be removed after the microneedle 120 is implanted into the skin. For example, in a state in which the microneedle 120 penetrates the skin, the user may apply force to remove the base 110 from the skin. To this end, the base 110 and the microneedle 120 may be detachably connected so that they may be separated when a predetermined external force is applied.

In another embodiment, in a state in which the microneedle patch 100 is disposed on the skin, a connection portion between the base 110 and the microneedle 120 may be melted or dissolved. For example, the connection portion between the base 110 and the microneedle 120 may be configured to melt when exposed to a temperature of body temperature for a predetermined time. Accordingly, the user may separate the base 110 from the microneedle 120. Alternatively, the connection portion between the base 110 and the microneedle 120 may be configured to melt or dissolve when exposed to room temperature or air for a predetermined time or longer. In another embodiment, the user may remove the base 110 by applying or spraying a material for dissolving the base 110.

In an embodiment, the base 110 may include any one of materials included in the microneedle 120. For example, the base 110 may include the same material as any one of a plurality of layers of the microneedle 120, and the material may be a biodegradable material.

In an embodiment, the base 110 may include a physiologically active substance. When the microneedle patch 100 is attached to the skin, an effective material EM of the microneedle 120 may be effectively delivered to a patient by the physiologically active substance emitted from the base 110. In addition, the base 110 and the microneedle 120 may be easily separated by the physiologically active substance emitted from the base 110.

In an embodiment, the base 110 may include a water-soluble polymer. For example, the base 110 may be made of the water-soluble polymer or may include other additives (e.g., disaccharides, etc.). In addition, the base 110 may not include the effective material EM or other effective materials.

In an embodiment, the base 110 may include a biocompatible material. The base 110 may include the same or different biocompatible material as a base material of the microneedle 120 to be described later.

The microneedle 120 may be inserted into the skin to directly administer a drug or an activity promoting material into the skin. The plurality of microneedles 120 may be disposed on one surface of the base 110 . The microneedle 120 may include the biocompatible material and an additive as a base material.

The biocompatible material includes at least one of carboxymethyl cellulose (CMC), alginic acid, pectin, carrageenan, chondroitin sulfate, dextran sulfate, chitosan, polylysine, carboxymethyl chitin, fibrin, agarose, pullulan, polyanhydride, polyorthoester, polyetherester, polyesteramide, polybutyric acid, polyvaleric acid, polyacrylate, ethylene-vinyl acetate Polymers, acryl-substituted cellulose acetate, polyvinyl chloride, polyvinyl fluoride, polyvinyl imidazole (polyvinyl), chlorosulphonate polyolefins, polyethylene oxide, polyvinylpyrrolidone (PVP), hydroxypropyl methylcellulose (HPMC), ethylcellulose (EC), hydroxypropyl cellulose (HPC), carboxymethyl cellulose, cyclodextrin, maltose, Lactose, trehalose, cellobiose, isomaltose, turanose and lactulose, or is one or more polymers selected from a group consisting of cellulose and copolymers of monomers forming such polymers.

The additive may include at least any one of trehalose, oligosaccharide, sucrose, maltose, lactose, cellobiose, hyaluronic acid, alginic acid, pectin, carrageenan, chondroitin sulfate, dextran sulfate, chitosan, polylysine, collagen, gelatin, carboxymethyl chitin, fibrin, agarose, PVP, polyethylene glycol (PEG), polymethacrylate, HPMC, EC, HPC, carboxymethyl cellulose, cyclodextrin, gentiobiose, cetrimide (alkyltrimethylammonium bromide), cetrimonium bromide (hexadecyltrimethylammonium bromide (CTAB)), gentian violet, benzethonium chloride, docusate sodium salt, a SPAN-type surfactant, polysorbate (Tween), sodium lauryl sulfate (sodium dodecyl sulfate (SDS)), benzalkonium chloride, and glyceryl oleate.

In one embodiment, hyaluronic acid (HA) may be excluded from the biocompatible material and the additives. If the activity promoting material is hyaluronidase, since the activity promoting material decomposes HA used as the base material, the performance of the microneedle 120 is reduced. Accordingly, when the microneedle 120 includes hyaluronidase as the activity promoting material, the base material does not include HA.

In an optional embodiment, the microneedle 120 may include an adhesive. The adhesive is at least one adhesive selected from a group consisting of silicone, polyurethane, HA, physical adhesive (gecko), polyacryl, ethyl cellulose, hydroxymethyl cellulose, ethylene-vinyl acetate and polyisobutylene.

In an optional embodiment, the microneedle 120 may additionally include metal, polymer or the adhesive.

The microneedle 120 may include the effective material EM and the activity promoting material APM. The microneedle 120 may include a pharmaceutical, medical, or cosmetically effective material EM in at least any part. As a non-limiting example, the effective material includes a protein/peptide medicine, but is not limited to, includes any one of a hormone, a hormone analogue, an enzyme, an enzyme inhibitor, a signal transduction protein or a portion thereof, an antibody or a portion thereof, a single-chain antibody, a binding protein or a binding domain thereof, antigens, adherent protein, structural proteins, regulatory proteins, toxic proteins, cytokines, transcription regulators, blood coagulation factors, and vaccines. More specifically, the protein/peptide medicine may include any one of insulin, insulinlikegrowth factor 1 (IGF-1), growth hormone, erythropoietin, granulocytecolony stimulating factors (G-CSFs), granulocyte/macrophage-colony stimulating factors (GM-CSFs), interferon alpha, interferon beta, interferon gamma, interleukin-1 alpha and beta, interleukin-3, interleukin-4, interleukin-6, interleukin-2, epidermal growth factors (EGFs), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II (GHRHII), gonadorelin, goserelin, histrelin, leuprorelin, lypressin, octreotide, oxytocin, pitressin, secretin ), sincalide, terlipressin, thymopentin, thymosine, triptorelin, bivalirudin, carbetocin, Cyclosporine, exedine, lanreotide, luteinizing hormone-releasing hormone (LHRH), nafarelin, parathyroid hormone, pramlintide, enfuvirtide (T-20), thymalfasin, and ziconotide. In addition, the effective material EM may be a cosmetic material such as skin lightening, filler, wrinkle reducing or antioxidant.

In one embodiment, the effective material EM may be a colloid dispersed in a solvent to form the microneedle 120 in the form of fine particles. The fine particles may be themselves the effective material EM, or may include a coating material carrying the effective material EM.

The effective material EM may be uniformly or non-uniformly distributed throughout the microneedle 120.

In another embodiment, the effective material EM may be dissolved in the microneedle 120. The effective material EM may be dissolved in the base material of the microneedle 120 such as the biodegradable materials described above to constitute the microneedle 120. The effective material EM may be dissolved in the base material at an even concentration, and may be intensively distributed at a specific height of the microneedle 120 like the above-mentioned fine particles.

In one embodiment, a pharmaceutical, medical, or cosmetically effective material EM may be coated on the microneedle 120. The effective material EM may be coated on the entire microneedle 120 or only a portion of the microneedle 120.

The activity promoting material APM is included in the microneedle 120 together with the effective material EM, so that the effective material EM may be effectively absorbed into the body. The activity promoting material APM includes various substances capable of increasing the absorption rate of the effective material EM, and in one embodiment, may be an enzyme that catalyzes the breaking of various chemical bonds.

In one embodiment, the activity promoting material APM may be any one of various enzymes that degrade extracellular matrix ECM. Accordingly, when the microneedle patch 100 is attached to the skin, the activity promoting material APM flows into the body from the microneedle 120 to decompose the extracellular matrix in the body, and the effective material EM may be effectively absorbed into the body. That is, the activity promoting material APM may increase the absorption rate of the effective material EM by decomposing the extracellular matrix in the body. For example, the activity promoting material APM may be hyaluronidase which is an enzyme that degrades or cuts HA as the extracellular matrix.

In an embodiment, at least one of mass per unit volume, moles per unit volume, and volume per unit volume of the activity promoting material APM may be smaller than the effective material EM. Here, the unit volume may mean the unit volume of an area in which the effective material EM and the activity promoting material APM are disposed in the microneedle patch 100, for example, the microneedle 120.

The mass per unit volume, the moles per unit volume and the volume per unit volume mean the mass, moles and volume of the effective material EM and the activity promoting material APM contained per the unit volume of the microneedle 120, respectively.

In one embodiment, in the total weight of the microneedle 120, the activity promoting material APM may occupy a greater weight than the effective material EM. For example, the weight of the effective material EM may be 5 to 20 times the weight of the activity promoting material APM. Preferably, the weight of the effective material EM may be 10 to 12 times the weight of the activity promoting material APM. If the weight of the effective material EM is less than 5 times that of the activity promoting material APM, the activity promoting material APM may excessively decompose the patient's extracellular matrix, so that the user's cells may be damaged, and the absorption of the effective material EM may be disturbed. If the weight of the effective material EM is greater than 20 times that of the activity promoting material APM, the activity promoting material APM may not decompose the HA of the extracellular matrix, so that the effective material EM is not effectively activated.

In an embodiment, the activity promoting material APM may be activated before the effective material EM. That is, in a state in which the microneedle 120 is inserted into the body, the activity promoting material APM may be delivered into the body before the effective material EM. Alternatively, in a state in which the activity promoting material APM and the effective material EM are delivered into the body, the activity promoting material APM decomposes the extracellular matrix in the body, especially HA, and then the effective material EM may react.

The microneedle 120 may have various shapes. The microneedle 120 may have a cone shape. For example, the microneedle 120 may have a polygonal shape such as a conical shape, a triangular pyramid shape, or a quadrangular pyramid shape.

The microneedle 120 may include one end connected to the base 110 and the other end having a sharpened tip ST inserted into the body. The microneedle 120 may have a shape in which a width is narrowed toward the sharpened tip ST.

The microneedles 120 may be arranged in various numbers and patterns. For example, as illustrated in FIG. 1, the microneedle 120 may be arranged in a plurality of rows and columns at the same interval. Alternatively, the plurality of microneedles 120 may be disposed at different intervals between adjacent microneedles 120.

The microneedle 120 may be formed of a single layer or a plurality of layers. In this embodiment, for convenience of description, a case in which the microneedle 120 is a single layer will be mainly described.

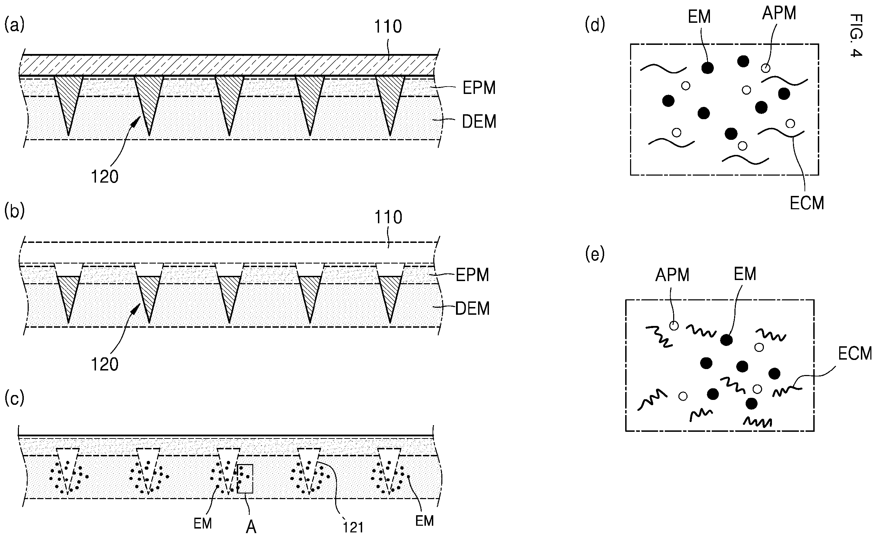

FIG. 4 is a diagram illustrating a process in which the microneedle patch 100 of FIG. 2 is attached and the effective material EM is delivered.

As illustrated in (a) of FIG. 4, when the microneedle patch 100 is attached to the skin, the microneedle 120 is inserted into the body. The depth into which the microneedle 120 is inserted or the position where the effective material EM and the activity promoting material APM are delivered is not particularly limited. FIG. 4 illustrates that the microneedle 120 penetrates an epidermis EPM and is inserted up to a dermis DEM to deliver the effective material EM and the activity promoting material APM into the dermis DEM, but the sharpened tip ST of the microneedle 120 may be located in the epidermis EPM, or the effective material EM and the activity promoting material APM may be delivered to the epidermis EPM. Alternatively, the sharpened tip ST of the microneedle 120 may be located in fat or muscle, or the effective material EM and the activity promoting material APM may be delivered to fat or muscle.

Next, as illustrated in (b) of FIG. 4, the base 110 may be decomposed or dissolved. For example, when a predetermined time elapses while the microneedle patch 100 is attached to the skin, the base 110 may be naturally decomposed. Alternatively, the base 110 may be melted by body temperature, or a separate material may be applied or sprayed to dissolve the base 110.

Next, as illustrated in (c) of FIG. 4, the effective material EM is delivered into the body from the microneedle 120.

More specifically, (d) of FIG. 4 illustrates an enlarged area A of (c) in which the effective material EM and the activity promoting material APM are released from the microneedle 120. The activity promoting material APM released from the microneedle 120 flows into the body and reacts with the extracellular matrix ECM (e.g., HA) in the body. And, as illustrated in (e) of FIG. 4, when the extracellular matrix ECM is decomposed by the activity promoting material APM, the effective material EM may be effectively delivered to the body.

In the microneedle patch 100 according to an embodiment of the present disclosure, the microneedle 120 includes the activity promoting material APM in addition to the effective material EM, thereby decomposing the extracellular matrix interfering with the delivery of the effective material EM, so that the effective material EM may be effectively delivered.

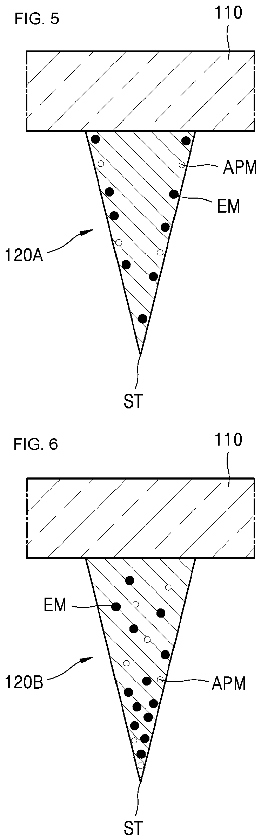

Each of FIGS. 5 to 16 is a diagram illustrating a modified example of FIG. 3.

Referring to FIG. 5, an effective material EM and/or an activity promoting material APM included in a microneedle 120A may be disposed in an outer region of the microneedle 120A. In more detail, the effective material EM and/or the activity promoting material APM may be disposed on an inside adjacent to a surface of the microneedle 120A. Here, an outside of the microneedle 120A may mean an outside of a central axis of the microneedle 120A passing through a sharpened tip ST. For example, the effective material EM and the activity promoting material APM may be disposed to be closer to an outer surface of the microneedle 120A than the central axis of the microneedle 120A. However, all of the effective material EM and the activity promoting material APM are not limited to being disposed only on the outside of the microneedle 120A, and at least some may be disposed in a center portion of the microneedle 120A.

As described above, in the state in which the microneedle 120A is inserted into the body, the effective material EM and the activity promoting material APM are intensively disposed in an area close to the body, so that the effective material EM and the activity promoting material APM may be delivered quickly and effectively into the body.

Referring to FIG. 6, an effective material EM and/or an activity promoting material APM included in a microneedle 120B may have a non-uniform concentration distribution. For example, the effective material EM and the activity promoting material APM may be disposed such that the concentration increases toward a sharpened tip ST along a height direction of the microneedle 120B. Accordingly, the effective material EM and the activity promoting material APM may be delivered quickly and effectively to a target site through the sharpened tip ST, which is inserted the deepest in the body.

Although not illustrated in the drawings, in another embodiment, the effective material EM and the activity promoting material APM may be disposed to increase in concentration toward the outer surface with respect to the central axis of the microneedle 120B. Therefore, it is possible to quickly and effectively deliver the effective material EM and the activity promoting material APM through the outer surface of the microneedle 120B having a large body contact area.

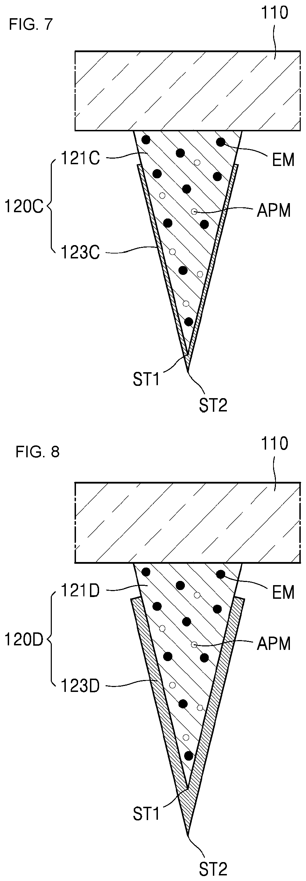

Referring to FIG. 7, a microneedle 120C may include a first needle portion 121C and a coating layer 123C.

The coating layer 123C may be disposed to surround an outer surface of the first needle portion 121C including a base material, an effective material EM, and an activity promoting material APM. For example, the coating layer 123C may cover a first sharpened tip ST1 of the first needle portion 121C, and may include a second sharpened tip ST2 corresponding to the first sharpened tip ST1. Here, a base material forming the coating layer 123C may be a second base material which is the same as or different from a first base material forming the first needle portion 121C.

After the first needle portion 121C is formed, it is immersed in a coating solution to form the coating layer 123C. The coating layer 123C may be formed of a biocompatible polymer, and may be decomposed after being inserted into the body.

In an embodiment, the coating layer 123C may include the activity promoting material. When the coating layer 123C is inserted into the skin, before the effective material EM of the first needle portion 121C is delivered, the activity promoting material of the coating layer 123C is first activated to increase the delivery effect of the effective material EM.

In an embodiment, the coating layer 123C may be made of a material having a high biodegradation rate. The coating layer 123C is formed of a material having a higher biodegradation rate than the first needle portion 121C, so that the coating layer 123C may be decomposed faster than the first needle portion 121C in a state in which the microneedle 120C is inserted into the body.

In another embodiment, the coating layer 123C may be made of a material having a low biodegradation rate. The coating layer 123C is formed of a material having a lower biodegradation rate than the first needle portion 121C, so that the effective material EM of the first needle portion 121C is not immediately delivered into the body while the microneedle 120C inserted into the body, and the effective material EM may be delivered into the body after a predetermined time elapses when the coating layer 123C is decomposed. That is, it is possible to deliver the effective material EM into the body at an appropriate target time.

In one embodiment, the coating layer 123C may increase the stiffness of the microneedle 120C. For example, the coating layer 123C may be made of a material having greater stiffness than the first needle portion 121C. Accordingly, when the microneedle 120C is inserted into the body, the coating layer 123C may protect the first needle portion 121C so that the first sharpened tip ST1 of the first needle portion 121C is not bent or broken.

In an embodiment, the first needle portion 121C may include the aforementioned biocompatible material and/or additive as the base material. In addition, the first needle portion 121C may include HA acid as the base material, and the coating layer 123C covering the outside of the first needle portion 121C may not include HA as the base material. That is, when the coating layer 123C includes hyaluronidase as the activity promoting material APM, when the microneedle 120C is inserted into the skin, the activity promoting material APM does not decompose the base material of the coating layer 123C, and a large amount of activity promoting material APM decomposes the extracellular matrix in the body. Thereafter, the effective material EM may be rapidly delivered into the body through the first needle portion 121C. At this time, even if the first needle portion 121C contains HA, it is not affected by the activity promoting material APM of the coating layer 123C.

Referring to FIG. 8, a coating layer 123D may have a predetermined thickness to include a larger amount of activity promoting material APM.

In an embodiment, the coating layer 123D may be formed of a second base material having a higher decomposition rate than a first base material constituting a first needle portion 121D. Accordingly, in a state in which a microneedle 120D is inserted into the body, a large amount of the activity promoting material APM decomposes the extracellular matrix in the body, and the coating layer 123D is decomposed, so that the effective material EM of the first needle portion 121D may be rapidly delivered into the body.

In one embodiment, the coating layer 123D may further include the effective material EM. That is, the coating layer 123D may include the second base material, the effective material EM and the activity promoting material APM.

In an embodiment, the first needle portion 121D may include the aforementioned biocompatible material and/or additive as the base material. In addition, the first needle portion 121D may include HA as the base material, and the coating layer 123D covering the outside of the first needle portion 121D may not include HA as the base material. That is, when the coating layer 123D includes hyaluronidase as the activity promoting material APM, when the microneedle 120D is inserted into the skin, the activity promoting material APM does not decompose the base material of the coating layer 123C, and a large amount of activity promoting material APM decomposes the extracellular matrix in the body. Thereafter, the effective material EM may be rapidly delivered into the body through the first needle portion 121D. At this time, even if the first needle portion 121D contains HA, it is not affected by the activity promoting material APM of the coating layer 123D.

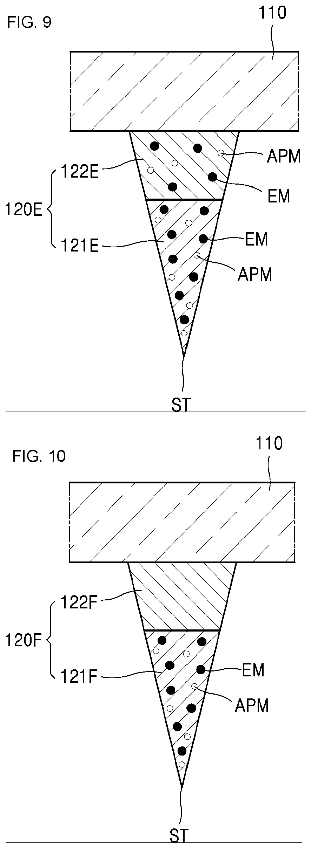

Referring to FIG. 9, a microneedle 120E may include a lateral structure by stacking a plurality of layers. For example, the microneedle 120E may include a first needle portion 121E and a second needle portion 122E.

The first needle portion 121E is disposed at a position most spaced apart from a base 110, and includes a sharpened tip ST at one end. The first needle portion 121E may include a first base material, an effective material EM and an activity promoting material APM.

The second needle portion 122E is disposed between the base 110 and the first needle portion 121E, and may include a second base material, the effective material EM, and the activity promoting material APM.

In one embodiment, in a state in which the microneedle 120E is inserted into the body, the first needle portion 121E penetrates the epidermis EPM and at least a part thereof may be inserted up to the dermis DEM, and the second needle portion 122E may be located in the epidermis EPM.

In an embodiment, the first base material and the second base material may have different decomposition rates. The decomposition rate of each layer may be determined according to the type and content of the biocompatible material constituting each layer.

In an embodiment, the second base material may have a higher decomposition rate than the first base material. Accordingly, the effective material EM may be rapidly delivered to the epidermis EPM. In addition, the second needle portion 122E connecting the base 110 and the first needle portion 121E is disassembled faster than the first needle portion 121E, so that the base 110 may be quickly removed, and the effective material EM included in the first needle portion 121E may be rapidly delivered to the skin.

In another embodiment, the second base material may have a lower decomposition rate than the first base material. Accordingly, the effective material EM may be rapidly delivered to the dermis DEM.

In one embodiment, the first needle portion 121E and the second needle portion 122E may have different stiffnesses. For example, the first needle portion 121E has greater stiffness than the second needle portion 122E, so that the first needle portion 121E may enter the body easily and quickly. In addition, when the first needle portion 121E is disassembled faster than the second needle portion 122E, pain may be minimized.

Although only two layers are illustrated in FIG. 9, the number of layers is not particularly limited. For example, in the microneedle 120E, three or more layers may be stacked in a height direction.

Referring to FIG. 10, a microneedle 120F includes a first needle portion 121F and a second needle portion 122F, and the second needle portion 122F may be made of only a second base material.

The second base material of the second needle portion 122F may have a higher decomposition rate than a first base material of the first needle portion 121F. In addition, since the second needle portion 122F is made of only the second base material, the effective material EM and the activity promoting material APM may be accurately deliver to a target position where the first needle portion 121F is inserted in the state in which the microneedle 120F is inserted into the skin (e.g., dermis DEM).

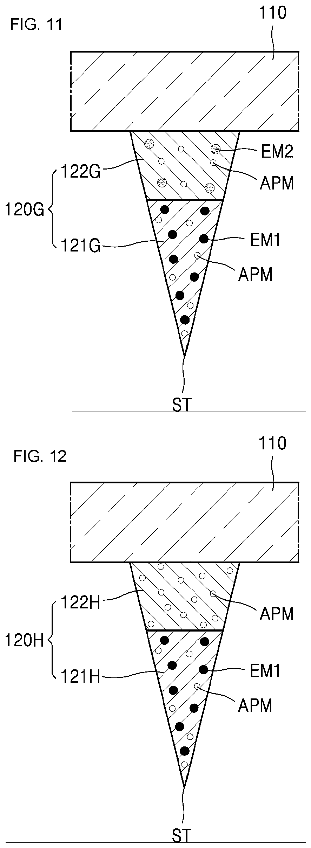

Referring to FIG. 11, a microneedle 120G includes a first needle portion 121G and a second needle portion 121G, and the second needle portion 122G is a second base material and a second effective material EM2. and an activity promoting material APM. Here, the second effective material EM2 may be a different drug from a first effective material EM1.

Accordingly, in a state in which the microneedle 120G is inserted into the body, the first effective material EM1 is delivered to an area corresponding to the first needle portion 121G, and the second effective material EM2 may be delivered to a body region corresponding to the second needle portion 122G. That is, the microneedle 120G may be inserted into the body to deliver different effective materials to different target positions.

The activity promoting material APM is contained in each of the first needle portion 121G and the second needle portion 122G, and may increase an absorption effect of the first effective material EM1 and the second effective material EM2 by decomposing the extracellular matrix in the body before each effective material is activated.

Referring to FIG. 12, a microneedle 120H includes a first needle portion 121H and a second needle portion 122H, and the second needle portion 122H may include a second base material and an activity promoting material APM.

The second needle portion 122H may contain a large amount of the activity promoting material APM instead of containing no effective material EM. In addition, a second base material of the second needle portion 122H may have a higher decomposition rate than a first base material of the first needle portion 121H.

Accordingly, while the microneedle 120H is inserted into the body, the second needle portion 122H is decomposed faster than the first needle portion 121H, and the large amount of activity promoting material APM contained in the second needle portion 122H may decompose the extracellular matrix in the body. Thereafter, an effective material EM of the first needle portion 121H is delivered into the body, thereby reducing the activation rate of the effective material EM.

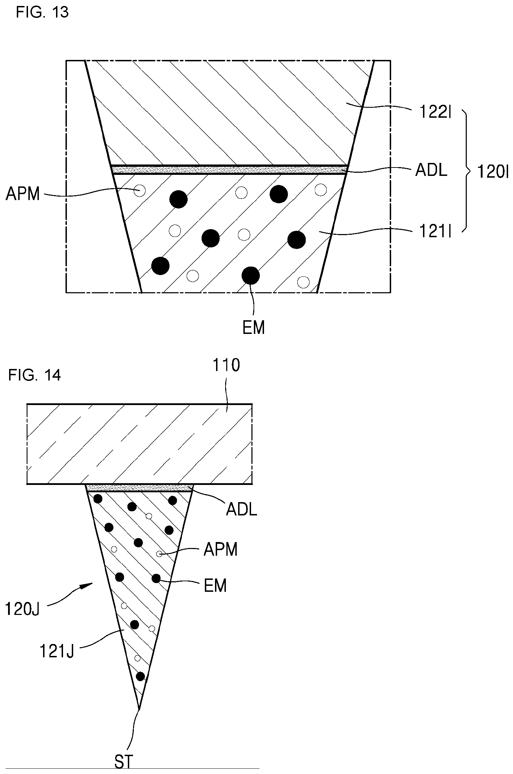

Referring to FIG. 13, a microneedle 1201 may include a first needle portion 121I, a second needle portion 1221, and an adhesive layer ADL.

The adhesive layer ADL may be disposed between the first needle portion 1211 and the second needle portion 1221, and may be formed of a material capable of increasing an adhesive force between the first needle portion 121I and the second needle portion 1221. Accordingly, when the microneedle 1201 is inserted into the skin, the first needle portion 1211 and the second needle portion 1221 may not be separated.

The adhesive layer ADL may be made of a biocompatible material or a biodegradable material. For example, the adhesive layer ADL may consist of a moisture layer.

In an embodiment, the adhesive layer ADL may be made of a material having a higher biodegradation rate than that of the first needle portion 121I and the second needle portion 1221. Accordingly, after the microneedle 1201 is inserted into the skin, the adhesive layer ADL is first decomposed, so that the first needle portion 121I and the second needle portion 1221 may be separated.

FIG. 13 illustrates that the adhesive layer ADL is disposed to partition a boundary between the first needle portion 121I and the second needle portion 1221, but is not limited thereto. For example, the adhesive layer ADL may have a form in which the first needle portion 121I and the second needle portion 1221 are mixed. A first base material of the first needle portion 1211 and a second base material of the second needle portion 1221 may be mixed to form the adhesive layer ADL.

Since the microneedle 1201 includes the adhesive layer ADL, coupling strength between the first needle portion 1211 and the second needle portion 1221 may be increased. Accordingly, when the microneedle 1201 is inserted into the skin, the first needle portion 1211 and the second needle portion 1221 are not easily separated by an external force applied to the microneedle patch. On the other hand, after the microneedle 1201 is inserted into the skin, the adhesive layer ADL is first decomposed, so that the first needle portion 1211 and the second needle portion 1221 may be separated.

Referring to FIG. 14, a microneedle 120J may include a first needle portion 121J and an adhesive layer ADL. The adhesive layer ADL may be disposed between a base 110 and the first needle portion 121J.

In one embodiment, the adhesive layer ADL may be made of a material having a higher biodegradation rate than the first needle portion 121J. Accordingly, when the microneedle 120J is inserted into the skin, the adhesive layer ADL is decomposed before the first needle portion 121J, and the base 110 may be separated.

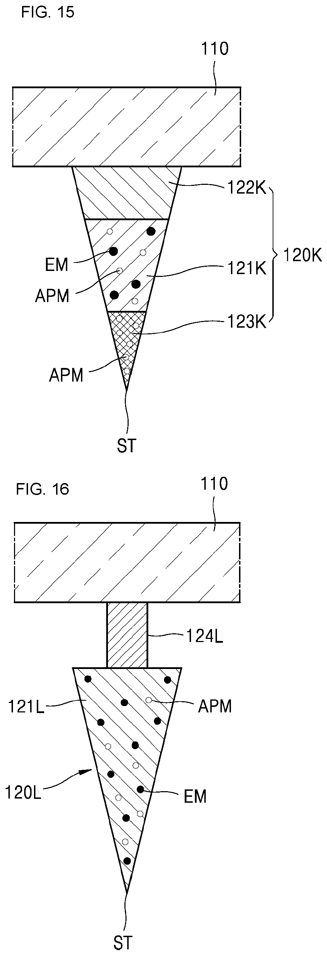

Referring to FIG. 15, a microneedle 120K may include a first needle portion 121K, a second needle portion 122K and a third needle portion 123K.

The first needle portion 121K includes a first base material, an effective material EM and an activity promoting material APM, and the second needle portion 122K is disposed between the base 110 and the first needle portion 121K, and may consist of a second base material. The third needle portion 123K may be disposed at one end of the first needle portion 121K to include a sharpened tip ST of the microneedle 120K.

In one embodiment, the third needle portion 123K may include the activity promoting material APM.

In one embodiment, in a state in which the microneedle 120K is inserted into the body, the third needle portion 123 may be decomposed faster than the first needle portion 121K and the second needle portion 122K.

Therefore, when microneedle 120K is inserted into the body, the activity promoting material APM may be activated as the third needle portion 123K is decomposed first. Afterwards, the effective material EM of the first needle portion 121K and the activity promoting material APM may be delivered into the body.

In one embodiment, the third needle portion 123K may be made of a material having greater stiffness than the first needle portion 121K and the second needle portion 122K. Therefore, when the microneedle 120K is inserted into the body, the sharpened tip ST may be inserted smoothly without being bent or damaged.

In one embodiment, the second needle portion 122K may be made of the second base material having a higher decomposition rate than the first base material of the first needle portion 121K. Therefore, when the microneedle 120K is inserted into the skin, the activity promoting material APM is activated as the third needle portion 123K is decomposed first, and then the second needle portion 122K is decomposed to separate the base 110 and the microneedle 120K. Next, the effective material EM and activity promoting material APM of the first needle portion 121K are delivered into the body.

Referring to FIG. 16, a microneedle 120L may include a first needle portion 121L and a shaft 124L.

The microneedle 120L may be applied to the microneedle of the above-described embodiments. However, hereinafter, for convenience of description, the microneedle 120 of FIG. 3 will be mainly described.

The first needle portion 121L is made of a base material, such as the aforementioned microneedle or needle portion, and may include an effective material EM and an activity promoting material APM.

The shaft 124L may connect a base 110 and the first needle portion 121L. The shaft 124L extends in a longitudinal direction of the first needle portion 121L, and may have a smaller diameter than an base 110 side end of the first needle portion 121L. The shape of shaft 124L is not particularly limited, and may have a cylindrical or polygonal column shape.

The microneedle 120L may be inserted deeper into the skin via the shaft 124L. That is, a sharpened tip ST of the first needle portion 121L is inserted at a deeper position by the shaft 124L, so that the effective material EM and the activity promoting material APM may be delivered deep into the skin. In addition, while the microneedle 120L is inserted into the skin, the base 110 side end of the first needle portion 121L functions as a stopper, thereby preventing the microneedle 120L from being easily separated from the skin until the shaft 124L is disassembled.

The shaft 124L is made of a biodegradable material and has a smaller volume than the first needle portion 121L, so it may be decomposed before the first needle portion 121L after being inserted into the skin. When shaft 124L is disassembled, the first needle portion 121L remains inserted into the skin, and the base 110 may be easily removed.

The shaft 124L may be made of a material having a higher decomposition rate than the first needle portion 121L. That is, the base material constituting the shaft 124L may be a material having a higher decomposition rate in vivo than the base material of the first needle portion 121L. Accordingly, when the microneedle 120L is inserted into the skin of a subject, the shaft 124L is rapidly dissolved so that the base 110 may be easily removed.

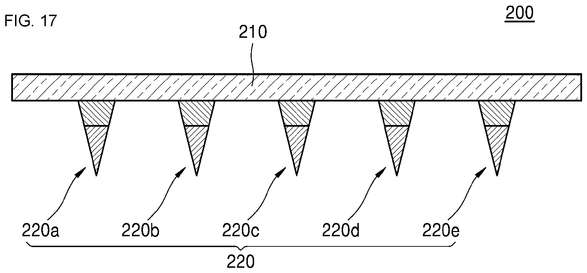

FIG. 17 is a view illustrating a microneedle patch 200 according to another embodiment of the present disclosure.

The microneedle patch 200 according to the present embodiment may include a plurality of microneedles 220. In addition, the plurality of microneedles 220 may be different from each other in at least one of shape, size, material, effective material EM and activity promoting material APM.

For example, the plurality of microneedles 220 may include a first microneedle 220a, a second microneedle 220b, a third microneedle 220c, a fourth microneedle 220d and a fifth microneedle 220e. In addition, the plurality of microneedles 220 may have different shapes, such as a cone, a triangular pyramid, or a quadrangular pyramid. Alternatively, the plurality of microneedles 220 may be made of a base material having different sizes or different decomposition rates. Alternatively, the plurality of microneedles 220 may include different effective material EM and/or different activity promoting material APM. Alternatively, the plurality of microneedles 220 may include the same type of effective material EM and/or activity promoting material APM, but may have different capacities.

Accordingly, microneedle patch 200 may deliver different effective material EM into the body, and may activate the effective material EM by using different activity promoting material APM depending on the effective material EM. In addition, the microneedle patch 200 may deliver different types or capacities of effective material EM depending on a location where it is attached to the skin.

As described above, the present disclosure has been described with reference to the embodiment shown in the drawings, but this is only an example. Those of ordinary skill in the art can fully understand that various modifications and equivalent other embodiments are possible from the embodiments. Therefore, the true technical protection scope of the present disclosure should be determined based on the appended claims.

Specific technical content described in the embodiment is an embodiment and does not limit the technical scope of the embodiment. In order to concisely and clearly describe the description of the disclosure, descriptions of conventional general techniques and configurations may be omitted. In addition, the connection or connection member of the lines between the components shown in the drawings exemplifies functional connections and/or physical or circuit connections, and in an actual device, various functional connections, physical connections that are replaceable or additional It may be expressed as a connection, or circuit connections. In addition, unless there is a specific reference such as "essential", "importantly", etc., it may not be a necessary component for the application of the present disclosure.

In the description and claims of the disclosure, "the" or similar referents may refer to both the singular and the plural unless otherwise specified. In addition, when a range is described in the embodiment, it includes the disclosure to which individual values belonging to the range are applied (unless there is a description to the contrary), and each individual value constituting the range is described in the description of the disclosure. same. In addition, the steps constituting the method according to the embodiment may be performed in an appropriate order unless the order is explicitly stated or there is no description to the contrary. The embodiments are not necessarily limited according to the order of description of the above steps. The use of all examples or exemplary terminology (e.g., etc.) in the embodiment is merely for describing the embodiment in detail, and unless it is limited by the claims, the scope of the embodiment is limited by the examples or exemplary terminology. it is not In addition, those skilled in the art will appreciate that various modifications, combinations, and changes may be made in accordance with design conditions and factors within the scope of the appended claims or their equivalents.

## Claims

1. A microneedle patch comprising:
a base; and
a microneedle disposed on a surface of the base, and including a base material, an effective material and an activity promoting material.

2. The microneedle patch of claim 1, wherein
the activity promoting material decomposes an extracellular matrix (ECM) to increase absorption rate of the effective material.

3. The microneedle patch of claim 1, wherein
the activity promoting material is activated prior to the effective material.

4. The microneedle patch of claim 1, wherein
the activity promoting material is disposed on an inside adjacent to the surface of the microneedle.

5. The microneedle patch of claim 1, wherein
the activity promoting material is disposed so that concentration thereof is increased from a center of the microneedle toward an outside.

6. The microneedle patch of claim 1, further comprising
a coating layer disposed on an outside of the microneedle, and including the activity promoting material.

7. The microneedle patch of claim 6, wherein
the microneedle comprises a first base material and the effective material,
the coating layer comprises a second base material different from the first base material and the activity promoting material, and
the second base material has a faster decomposition rate than the first base material.

8. The microneedle patch of claim 1, wherein
at least one of mass per unit volume, moles per unit volume, and volume per unit volume of the activity promoting material is smaller than that of the effective material.

9. The microneedle patch of claim 1, wherein
the activity promoting material is hyaluronidase.

10. The microneedle patch of claim 1, wherein
the microneedle comprises
a first needle portion including a first base material, a first effective material and the activity promoting material, and
a second needle portion disposed between the base and the first needle portion, and including a second base material, a second effective material and the activity promoting material.

11. The microneedle patch of claim 1, wherein
the microneedle comprises
a first needle portion including a first base material, a first effective material and the activity promoting material, and
a second needle portion disposed between the base and the first needle portion, and including a second base material and the activity promoting material, and
the second base material is a faster decomposition rate than the first base material.

12. The microneedle patch of claim 1, wherein
the microneedle comprises
a first needle portion including a first base material, a first effective material and the activity promoting material, and
a second needle portion disposed between the base and the first needle portion, and including a second base material and a second effective material, and
the first base material has a faster decomposition rate than the second base material.
